# EUROPEAN PATENT SPECIFICATION

(11) **EP 1 555 041 B1**
(45) Date of publication and mention of the grant of the patent: **13.12.2006**
(21) Application number: 04023328.0
(22) Date of filing: 30.09.2004
(51) Int. Cl.: A61M 39/02

(54) **Needleless injection adapter**
Nadelloser Injektionsadapter
Adaptateur d'injection sans aiguille

(30) Priority: 14.01.2004 JP 2004007348
(43) Date of publication of application: 20.07.2005
(73) Proprietor: Orchis Company Limited, Tokyo 104-0033 (JP)
(72) Inventor: Minezaki, Susumu, c/o Orchis Company Limited, Tokyo 104-0033 (JP)
(74) Representative: Volpert, Marcus

(56) References cited:
- EP-A- 0 642 805
- US-A- 4 311 136
- US-A- 4 645 496

## Description

### BACKGROUND OF THE INVENTION

### 1. Field of the Invention

The present invention relates to a needleless injection adapter which is disposed in the middle of an extracorporeal blood circuit used for treatment for renal failure and the like, or with a blood or fluid transfusion set, in order to inject medical fluid into a patient or collect blood from a patient with the extracorporeal blood circuit at appropriate time.

### 2. Description of the Related Art

A conventional injection adapter has a duct body connected to fluid conveying tubes at its both ends and having an erect tube provided in a side face thereof. On the top face of the erect tube a rubber plug with a portion thereof exposed is fixed by a cap. This injection adapter is disposed at some position of the fluid conveying tube of an extracorporeal blood circuit or a blood or fluid transfusion set. When necessary, medical fluid is injected by use of an injector or the like by sticking an injection needle to an exposed surface of the rubber plug.

In the foregoing injection adaptor, the injection needle is pulled out of the rubber plug after the medical fluid injection. There is a possibility that in the process of detaching the injection needle from the injector for segregated discarding, an operator may accidentally stick a tip of the needle into his/her finger, and may get infected from infectious blood adhering to the needle.

The inventor of the present invention has previously proposed a new needleless injection adapter which can inject medical fluid by use of an injector without an injection needle. The needleless injection adapter according to this new proposal has been disclosed in Japanese Unexamined Patent Application Publication No. 2003-369886. In the needleless injection adapter, as shown in Fig. 5, a duct body 52 has connecting portions to connect with fluid conveying tubes 51 at both ends thereof. A rotatable member 56 having a socket 55, to which an injector 54 is coupled, is slidably provided in a side face 53 of the duct body 52. When the rotatable member 56 is rotated by a predetermined angle, a small hole 58 extending to the socket 55 of the rotatable member 56 is aligned with a small hole 57 formed in a side face of the duct body 52 to extend therethrough. Thus, injection is carried out by coupling a tip (without a needle) 54a of the injector 54 to the socket 55 erected on the rotatable member 56, and rotating the rotatable member 56.

The needleless injection adapter according to the foregoing new proposal is advantageous because injection is carried out by use of the injector without an injection needle. However, when the tip end 54a of the injector 54 is coupled to the socket 55 erected on the rotatable member 56, to rotate the rotatable member 56 together with the injector, thereby aligning the small holes 57 and 58, an operator is not able to surely know the alignment of the small holes. Thus, the operator cannot know whether or not the injection is normally done. Also, it is troublesome for the operator to return the angle of the rotatable member 56 to an angle with which the small holes 57 and 58 are misaligned with each other, before pulling the tip 54a of the injector 54 out of the socket 55 erected on the rotatable member 56 after the injection.

Furthermore, when the socket 55 of the rotatable member 56 is coupled to a continuous medical fluid injector for continuous medical fluid injection of long hours, for example, it is necessary to continuously retain the alignment of the small holes 57 and 58; however, the needleless injection adapter according to the foregoing new proposal is not provided with position retaining means (especially the needleless injection adapter with a spring).

A needleless injection adapter according to the preamble of claim 1 is known from EP 0 642 805 A2.

### SUMMARY OF THE INVENTION

In view of solving all of various problems of the needleless injection adapter according to the foregoing new proposal, an object of the present invention is to provide a needleless injection adapter which allows an operator to normally perform injection with safety, and automatically brings small holes into a misalignment after the injection.

Another object of the present invention is to provide a needleless injection adapter with position retaining means which can continuously retain the alignment of the small holes when necessary.

In view of achieving the above objects, the needleless injection adapter according to the present invention includes the features mentioned in claim 1. As configured above, the operator is able to confirm with sureness that the injection is normally done.

Other embodiments of the present invention are mentioned in the subclaims.

The needleless injection adapter according to the present invention is configured to include a duct body and a rotatable member having a socket for an injector and being slidably in contact with a side face of the duct body, so that a first small hole extending through the side face of the duct body and a second small hole extending to the socket of the rotatable member are aligned with each other when the rotatable member is rotated by a predetermined angle. The needleless injection adapter is configured to further include: a spring member biasing the socket in a direction where the first and second holes are misaligned with each other; and a sound emission member and/or a tactile member being provided in the socket, snapping a tip end of the spring member at a position where the first and second holes are aligned with each other. Such a configuration makes it possible to align the small hole extending through the side face of the duct body with another small hole extending to the socket of the rotatable member, by coupling the socket erected on the rotatable member to the tip of the injector without a needle to rotate the rotatable member, whereby the operator is surely informed of the alignment point with a sound or an impact by snapping the tip end of the spring member.

Therefore, it is advantageous that the operator is able to smoothly and securely carry out the injection since he or she can operate the injector with the rotatable member in rotation upon hearing the sound or impact. Also, when the operator loosens his/her hold after the injection, the rotatable member is automatically returned to a position in which the small holes are misaligned with each other, due to the action of the spring member. This eliminates the need for the operator to return the rotatable member to the misalignment position of the small holes by himself/herself. This also enables the operator to pull the tip of the injector out of the socket at ease. Accordingly, the needleless injection adapter can provide various beneficial effects.

According to the present invention, the sound emission member and/or the tactile member include(s) the tip end of the spring member whose base end is held by the duct body, and a projection or a recess on the side face of the socket with which the tip end thereof is in contact. The sound or impact is emitted when the tip end of the spring member makes contact with the projection or the recess on the side face of the socket.

Thus, it is possible to make the emission of the sound or the impact coincide with the alignment of the small holes, so that the operator is beneficially informed of the alignment of the small holes with precision.

According to another aspect of the needleless injection adapter of the present invention, the needleless injection adapter is provided with a position retaining member which retains the rotatable member against the spring force of the spring member in such a position that the small holes are aligned with each other. For example, the position retaining member in the rotatable member can continuously retain the alignment of the small holes during continuous medical fluid injection, if medical fluid is continuously injected for many hours with use of a continuous medical fluid injector coupled to the socket of the rotatable member. Also, without the position retaining member, the action of the spring member automatically misaligns the small holes. Therefore, it is advantageous that the needleless injection adapter is connected to and disconnected from the continuous medical fluid injector at ease.

### BRIEF DESCRIPTION OF THE DRAWINGS

The nature, principle, and utility of the invention will become more apparent from the following detailed description when read in conjunction with the accompanying drawings in which like parts are designated by identical reference numbers, in which:
Fig. 1 is a perspective view of a needleless injection adapter according to the present invention;
Fig. 2 is an enlarged sectional view of the needleless injection adapter according to the present invention;
Fig. 3 is a sectional view of the needleless injection adapter according to the present invention, in which small holes are misaligned with each other;
Fig. 4 is a sectional view of the needleless injection adapter according to the present invention, in which the small holes are aligned with each other; and

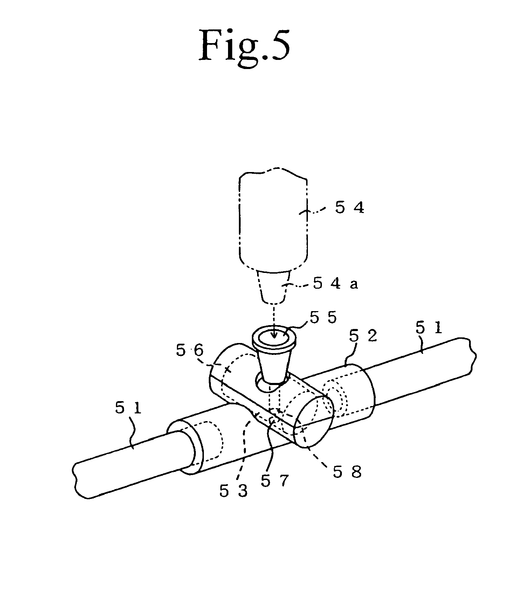

Fig. 5 is a perspective view showing prior art needleless injection adapter proposed by the inventor of the present invention.

### DESCRIPTION OF THE PREFERRED EMBODIMENTS

In the following the embodiments of the invention will be described with reference to the drawings.

In the needleless injection adapter 1 according to the present embodiment, a rotatable member 6 having a socket 5, to which a tip 4a of an injector 4 is coupled, is slidably disposed in a side face 3 of a duct body 2. When the rotatable member 6 is rotated by a predetermined angle (for example, the rotatable member 6 becomes orthogonal to the duct body 2), a small hole 8 extending to the socket 5 of the rotatable member 6 is aligned with a small hole 7 which is formed in the side face 3 of the duct body 2 to extend therethrough.

The rotatable member 6 refers to what is rotatably contained in a duct 9 which is fixed over the duct body 2 in an orthogonal manner. Thus, the side face 3 of the duct body 2 corresponds to the bottom face of the orthogonal duct 9. The small hole 7 is formed at one position (in the center) on the bottom face of the orthogonal duct 9, and extends through the side face 3 of the duct body 2. Furthermore, the socket 5 erected on the rotatable member 6 protrudes to outside from a through hole 10 which is formed in the top wall of the orthogonal duct 9 along its circumference.

To secure the air tightness between the small hole 7 extending through the side face 3 of the duct body 2 and the small hole 8 extending to the socket 5 of the rotatable member 6, a packing member 6b may be fitted into a notch groove 6a formed in the bottom face of the rotatable member 6, and the small hole 8 may extend and penetrate the packing member 6b. In this case, if necessary, means may be provided for preventing the leakage of fluid from a contact surface between the small hole 8 in the rotatable member 6 and the small hole 8 in the packing member 6b, such as a projection (not illustrated) in the shape of O-ring to surround the periphery of the small hole 8 in the rotatable member 6. The projection in the shape of O-ring may also surround the periphery of the small hole 7 in the duct body 2, to function as leakage prevention means during the alignment of the small hole 7 with the small hole 8 in the packing member 6b.

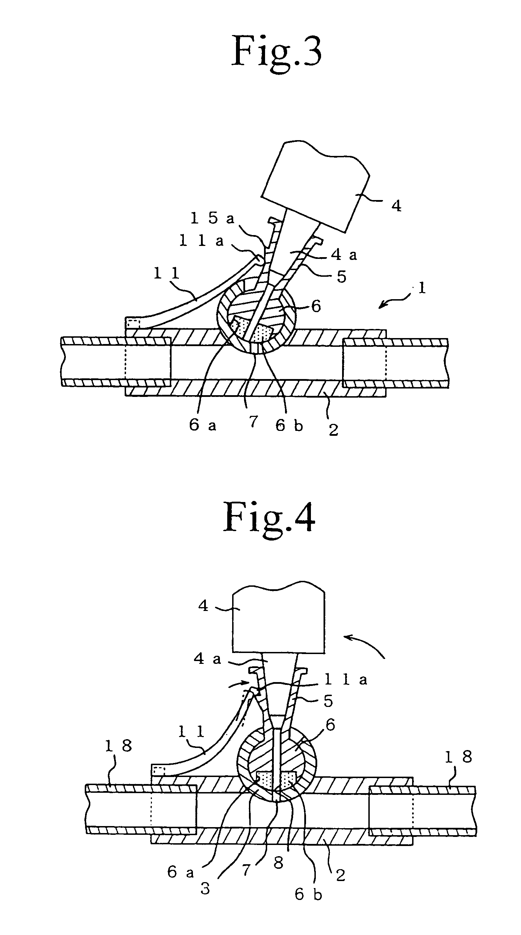

The side face of the socket 5 erected on the rotatable member 6 is biased and inclined by the tip end 11a of a spring member 11 whose base end is held by the duct body 2. Thus, the small holes 7 and 8 are misaligned from each other in a normal state (refer to Fig. 3). The base end of the spring member 11 extends from ring-shaped members 12 fitted over both end portions of the orthogonal duct body 9. The base end of the spring member 11 is integral with a base frame 14 supported by pins 13, 13a, and 13b which stand near an end of the duct body 2. The ring-shaped members 12 integral with the base frame 14 are conducive to the coalescence between the divided two portions of the orthogonal duct 9, the bottom face portion a and the top face portion b.

The socket 5 of the rotatable member 6 is provided with a sound emission member and/or tactile member 15 which snaps the tip end 11a of the spring member 11 to emit a sound or an impact at an alignment position of the small holes 7 and 8. The sound emission member 15 is composed of the tip end 11a of the spring member 11 whose base end is held by the duct body 2, and a projection 15a provided on the side face of the socket 5 making contact with the tip end 11a. Namely, the tip 4a of the injector 4 without a needle is coupled to the socket 5 erected on the rotatable member 6 to rotate the rotatable member 6 with the injector 4, thereby aligning the small holes 7 and 8 with each other. At this time, held by the duct body 2 at the base end, the tip end 11a of the spring member 11 falls down from the top of the projection 15a provided on the side face of the socket 5, and is snapped down to tap the side face of the socket 5, emitting a sound or an impact.

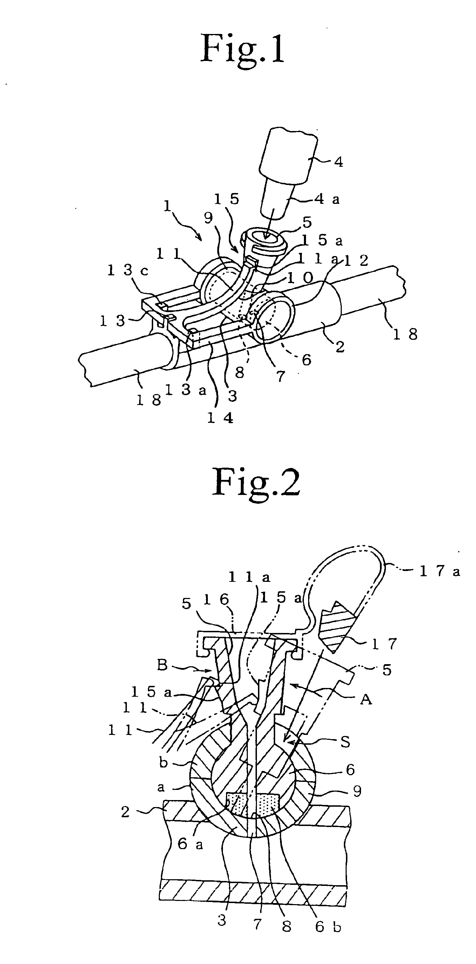

In other words, when the socket 5 with the injector 4 coupled thereto is rotated from a position illustrated by chain double-dashed lines to a position illustrated by solid lines in the direction of the arrow A in Fig. 2, the tip end 11a of the spring member 11 runs up on a projection 15a and falls down from the top portion thereof in the direction of the arrow B. The tip end 11a of the spring member 11 is snapped by the projection 15a to tap the side face of the socket 5, emitting a sound or an impact. Instead of the projection 15a, a recess (not illustrated) may be provided in the side face of the socket 5. In this case, the tip end 11a of the spring member 11 falls down into the recess from the end portion of the recess and is snapped.

The socket 5 of the rotatable member 6 of the needleless injection adapter 1 may be provided with a cap 16 which is put on the socket 5 while the needleless injection adapter 1 is in no use. The cap 16 is used as necessary for preventing invasion of bacteria, dust, and the like into the socket 5. As a matter of convenience, it is preferably configured such that sterilizing fluid can flow through the socket 5 with the cap 16 put on during the sterilization of the needleless injection adapter 1, and after the sterilization the socket 5 is completely sealed by, for example, strongly pressing the cap 16 into the socket 5.

Being coupled to a continuous medical fluid injector (not illustrated), the needleless injection adapter 1 may be used for continuous medical fluid injection of long hours. In this case, it is necessary to retain an alignment between the small holes 7 and 8 by rotating the rotatable member 6 against the spring force of the spring member 11. The needleless injection adapter 1 is provided with a position retaining member 17, as an accessory for this purpose, which allows rotation of the rotatable member 6 against the spring force of the spring member 1 to retain an alignment position of the small holes 7 and 8. The structure of the position retaining member 7 is not specifically limited as long as the position retaining member 17 can retain the rotational position of the rotatable member 6 while the small holes 7 and 8 are aligned with each other. Here, the position retaining member 17 is a wedge-shaped member shown by chain double-dashed lines which is inserted into the clearance S occurring between the duct body 2 and the rotatable member 6 when the small holes 7 and 8 are aligned with each other. Needless to say, the wedge-shaped member has to be configured not to be easily pulled out from the clearance S when once inserted thereinto, except that the operator pulls it out on purpose.

In this embodiment, as shown in Fig. 2, the wedge-shaped member as an example of the position retaining member 17 is formed in the end of a line member 17a extending from the cap 16 put on the socket 5 of the rotatable member 6. The wedge-shaped member, however, may be formed in another member, for example, in a line member (not illustrated) extending from the base frame 14 of the spring member 11. The wedge-shaped member may be independent from the cap 16.

In the following, the operation of the needleless injection adapter 1 will be described with reference to Figs. 3 and 4. First, both ends of the duct body 2 of the needleless injection adapter 1 are connected to the ends of the fluid conveying tubes 18 of an extracorporeal blood circuit (not illustrated). In the needleless injection adapter 1 according to the present embodiment, as shown in Fig. 3, the socket 5 of the rotatable member 6 is obliquely biased by the tip end 11a of the spring member 11 at first. In other words, the small hole 8 extending to the socket 5 of the rotatable member 6 is misaligned with the small hole 7 extending through the side face 3 of the duct body 2. In this state, blood flows from the fluid conveying tubes 18 through the duct body 2 without backflowing through the small holes 7 and 8, so that dialysis treatment and the like are carried out at ease.

When it becomes necessary to inject medical fluid into a patient as shown in Fig. 3, the tip 4a of the injector 4 without a needle is coupled to the socket 5 erected on the rotatable member 6. Then, as shown in Fig. 4, the socket 5 with the injector 4 is rotated against the spring force of the spring member 11 in a vertical direction (in the direction of the arrow). In this case, the tip end 11a of the spring member 11 runs up along the projection 15a on the side face of the socket 5. Falling from the top portion of the projection 15a, the snapped tip end a of the spring member 11 emits a sound or an impact (the operation of the sound emission member 15). A time when the sound or impact is emitted, that is, when the sound emission member 15 operates coincides with the time when the small holes 7 and 8 get aligned with each other, so that upon hearing the sound or impact, an operator clearly confirm the alignment of the small holes 7 and 8.

Then, the operator pushes a plunger while vertically holding the socket 5 with the injector 4, to inject medical fluid into the patient through the fluid conveying tubes 18 with blood flowing through the duct body 2. Upon completing this injection operation, the operator loosens his/her vertical hold of the injector 4, allowing it to rotate to be oblique by the spring force of the spring member 1 as shown in Fig. 3. Therefore, the small holes 7 and 8 are brought into the non-communication state. Thereafter, the tip 4a of the injector 4 is pulled out of the socket 5. The patient is connected to the extracorporeal blood circuit (the blood or fluid transfusion set) to continue the original treatment. Accordingly, it is possible to complete a series of medical fluid injection operation with smoothness and with safety.

The needleless injection adapter 1 according to the present embodiment is also applied for continuous medical fluid injection of long hours by coupling a medical fluid continuous injector (not illustrated) to the socket 5 of the rotatable member 6. Namely, with the alignment of the small hole 8 and the small hole 7, as shown in Fig. 2, the wedge-shaped clearance S is formed between the duct body 2 and the rotatable member 6. Inserting an wedge-shaped member 17 into the clearance S makes it possible to continuously retain the alignment of the small holes 7 and 8.

## Claims

1. Needleless injection adapter comprising
a duct body (2) having a first small hole (7) extending through a side face (3) thereof,
a rotatable member (6) being slidably in contact with said side face (3) of said duct body (2), said rotatable member (6) having a socket (5) for an injector (4) and a second small hole (8) extending to said socket (5),
said first small hole (7) and said second small hole (8) being aligned with each other when the rotatable member (6) is rotated by a predetermined angle,
**characterized by**
a spring member (11) biasing said socket (5) of said rotatable member (6) into such a direction that said first and second holes (7, 8) are misaligned to each other, and
a sound emission member and/or a tactile member (15) being composed of a tip end (11a) of said spring member (11) and a projection portion (15a) or a recess portion provided on that side face of said socket (5) making contact with said tip end (11a), said projection portion (15a) or recess portion and said tip end (11 a) of the spring member (11) being arranged so as to emit sound or give impact when said rotatable member (6) is rotated to a position where said first and second holes (7, 8) are aligned with each other and said tip end (11a) is snapped by the projection portion (15a) or recess portion to tap said side face of said socket (5).

2. Needleless injection adapter according to claim 1, **characterized in that** said projection portion (15a) or recess portion and said tip end (11a) are arranged so that the tip end (11a) of said spring member (11) runs up on said projection portion (15a) and falls down from the top portion thereof or falls down into said recess portion when the socket (5) with the injector (4) coupled thereto is rotated to a position where said first and second holes (7, 8) are aligned with each other.

3. Needleless injection adapter according to claim 1 or 2, **characterized by** a position retaining member (17) which retains said rotational member (6) in a rotational position where said first and second holes (7, 8) are aligned with each other.

4. Needleless injection adapter according to claim 3, **characterized in that** a clearance (S) is provided between the duct body (2) and said rotational member (6), and the position retaining member (17) is a wedge-shaped member which may be inserted into said clearance (S) in order to retain said rotational member (6) **in that** rotational position where said first and second holes (7, 8) are aligned with each other.

## Patentansprüche

1. Nadelloser Injektionsadapter mit einem Kanalkörper (2), der ein erstes kleines Loch (7) aufweist, das sich durch eine Seitenfläche (3) des Kanalkörpers erstreckt, einem drehbaren Teil (6), der mit der Seitenfläche (3) des Kanalkörpers (2) in gleitender Berührung steht, wobei der drehbare Teil (6) eine Fassung (5) für einen Injektor (4) aufweist und mit einem zweiten kleinen Loch (8) versehen ist, das sich durch die Fassung (5) erstreckt, wobei das erste kleine Loch (7) und das zweite kleine Loch (8) miteinander fluchten, wenn der drehbare Teil (6) um einen vorbestimmten Winkel gedreht wird, **gekennzeichnet durch** einen Federkörper (11), der die Fassung (5) des drehbaren Teils in eine solche Richtung drückt, daß die ersten und zweiten Löcher (7, 8) nicht miteinander fluchten, und **durch** einen geräuschaussendenden Körper und/oder einen Tastkörper (15), der aus einem spitzen Ende (11a) des Federkörpers (11) und einem Vorsprungabschnitt (15a) oder einem Vertiefungsabschnitt auf der Seitenfläche der Fassung (5) gebildet wird, die mit dem spitzen Ende (11a) in Berührung tritt, wobei der Vorsprungabschnitt (15a) oder der Vertiefungsabschnitt und das Spitzenende (11a) des Federkörpers (11) so angeordnet sind, daß ein Geräusch ausgesandt oder ein Stoß abgegeben wird, sobald der drehbare Teil (6) in eine Lage gedreht wird, in der die ersten und zweiten Löcher (7, 8) miteinander fluchten und das spitze Ende (11a) **durch** den Vorsprungabschnitt (15a) oder Vertiefungsabschnitt einschnappt, um die Seitenfläche der Fassung (5) aufzunehmen.

2. Nadelloser Injektionsadapter nach Anspruch 1, **dadurch gekennzeichnet, daß** der Vorsprungabschnitt (15a) oder Vertiefungsabschnitt und das Spitzenende (11a) so angeordnet sind, daß das spitze Ende (11a) des Federkörpers (11) auf dem Vorsprungabschnitt (15a) läuft und von seinem oberen Abschnitt herabfällt oder in den Vertiefungsabschnitt herabfällt, sobald die Fassung (5) mit dem angekoppelten Injektor (4) in eine Lage gedreht wird, in der die ersten und zweiten Löcher (7, 8) miteinander fluchten.

3. Nadelloser Injektionsadapter nach Anspruch 1 oder 2, **gekennzeichnet durch** einen Positionshaltekörper (17), der den drehbaren Teil (6) in einer Drehstellung hält, in der die ersten und zweiten Löcher (7, 8) miteinander fluchten.

4. Nadelloser Injektionsadapter nach Anspruch 3, **dadurch gekennzeichnet, daß** zwischen dem Kanalkörper (2) und dem drehbaren Teil (6) ein Spalt (S) vorgesehen ist, und daß der Positionshaltekörper (17) ein keilförmiger Körper ist, der in diesen Spalt (S) eingesteckt werden kann, um den drehbaren Teil (6) in derjenigen Drehstellung zu halten, in der die ersten und zweiten Löcher (7, 8) miteinander fluchten.

## Revendications

1. Adaptateur d'injection sans aiguille, comprenant :
un corps de conduit (2) ayant un premier petit trou (7) s'étendant à travers une face latérale (3) de celui-ci,
un élément rotatif (6) qui est en contact coulissant avec ladite face latérale (3) dudit corps de conduit (2), ledit élément rotatif (6) ayant un socle (5) pour un injecteur (4) et un second petit trou (8) s'étendant jusqu'audit socle (5),
ledit premier petit trou (7) et ledit second petit trou (8) étant alignés l'un avec l'autre quand l'élément rotatif (6) est tourné d'un angle prédéterminé,
**caractérisé par**
un élément à ressort (11) qui repousse ledit socle (5) dudit élément rotatif (6) dans une direction de telle que ledit premier et ledit second trou (7, 8) ne sont pas alignés l'un avec l'autre, et
un élément d'émission sonore et/ou un élément tactile (15) qui est composé d'une extrémité d'embout (11a) dudit élément à ressort (11) et d'une portion en projection (15a) ou d'une portion évidée prévue sur la face latérale dudit socle (5) qui entre en contact avec ladite extrémité d'embout (11a), ladite portion en projection (15a) ou ladite portion évidée et ladite extrémité d'embout (11a) de l'élément à ressort (11) étant agencées de manière à émettre un son ou à produire un déclic quand ledit élément rotatif (6) est tourné à une position à laquelle ledit premier trou et ledit second trou (7, 8) sont alignés l'un avec l'autre, et
ladite extrémité d'embout (11a) est engagée en encliquetage par la portion en projection (15a) ou par la portion évidée pour venir en prise avec ladite face latérale dudit socle (5).

2. Adaptateur d'injection sans aiguille selon la revendication 1, **caractérisé en ce que** ladite portion en projection (15a) ou ladite portion évidée et ladite extrémité d'embout (11a) sont agencées de telle façon que l'extrémité d'embout (11a) dudit élément à ressort (11) se déplace en montant sur ladite portion en projection (15a) puis retombe depuis la portion supérieure de celle-ci ou tombe dans ladite portion évidée quand le socle (5) avec l'injecteur (4) couplé à lui-même est tourné à une position à laquelle ledit premier trou et ledit second trou (7, 8) sont alignés l'un avec l'autre.

3. Adaptateur d'injection sans aiguille selon la revendication 1 ou 2, **caractérisé par** un élément de retenue en position (17) qui retient ledit élément rotatif (6) dans une position de rotation dans laquelle ledit premier trou et ledit second trou (7, 8) sont alignés l'un avec l'autre.

4. Adaptateur d'injection sans aiguille selon la revendication 3, **caractérisé en ce qu'**il est prévu un jeu (S) entre le corps de conduit (2) et ledit élément rotatif (6), et **en ce que** l'élément de retenue en position (17) est un élément en forme de coin qui peut être inséré dans ledit jeu (S) afin de retenir ledit élément rotatif (6) dans la position en rotation dans laquelle ledit premier trou et ledit second trou (7, 8) sont alignés l'un avec l'autre.
